(19) Europäisches Patentamt
European Patent Office
Office européen des brevets

(11) EP 1 135 095 B1

# (12) FASCICULE DE BREVET EUROPEEN

(45) Date de publication et mention de la délivrance du brevet:
**08.12.2004 Bulletin 2004/50**

(21) Numéro de dépôt: **00966245.3**

(22) Date de dépôt: **29.09.2000**

(51) Int Cl.$^7$: **A61K 7/06**

(86) Numéro de dépôt international:
**PCT/FR2000/002714**

(87) Numéro de publication internationale:
**WO 2001/024767 (12.04.2001 Gazette 2001/15)**

## (54) COMPOSITION COSMETIQUE COMPRENANT AU MOINS UN CATION, UN ALCOOL GRAS LIQUIDE ET AU MOINS UN COMPOSE DE TYPE CERAMIDE ET PROCEDE

KOSMETISCHE ZUSAMMENSETZUNG, DIE MINDENSTENS EIN KATION, EINEN FLÜSSIGEN FETTALKOHOL UND EINE VERBINDUNG DES TYPS CERAMID ENTHÄLT, UND VERFAHREN

COSMETIC COMPOSITION COMPRISING AT LEAST A CATION, A LIQUID FATTY ALCOHOL AND AT LEAST A CERAMIDE TYPE COMPOUND AND METHOD USING SAME

(84) Etats contractants désignés:
**AT BE CH CY DE DK ES FI FR GB GR IE IT LI LU MC NL PT SE**

(30) Priorité: **07.10.1999 JP 28741299**

(43) Date de publication de la demande:
**26.09.2001 Bulletin 2001/39**

(73) Titulaire: **L'OREAL**
**75008 Paris (FR)**

(72) Inventeurs:
- **YAMADA, Shinichi**
**Yokohama (JP)**
- **BONE, Eric**
**F-92110 Clichy (FR)**

(74) Mandataire:
**Le Blainvaux Bellegarde, Françoise et al**
**L'OREAL - D.I.P.I.**
**25-29 Quai Aulagnier**
**92600 Asnières (FR)**

(56) Documents cités:
**EP-A- 0 278 505**
**WO-A-99/29293**
**FR-A- 2 679 770**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 135 095 B1

Printed by Jouve, 75001 PARIS (FR)

## Description

La présente invention a trait à une composition cosmétique pour le traitement des matières kératiniques telles que les cheveux, comprenant au moins un tensioactif cationique, au moins un alcool gras liquide et au moins un composé de type céramide ainsi qu'au procédé de traitement non-thérapeutique à l'aide de cette composition.

On connaît déjà dans l'état de la technique des formulations capillaires permettant de traiter les cheveux abîmés par les intempéries ou les traitements capillaires physiques (brushing, peignage ...) ou chimiques (coloration, permanente...).

L'un des moyens couramment utilisés pour améliorer le démêlage et la douceur de ces cheveux consiste à utiliser des compositions de soin, puis à rincer les cheveux à l'eau. En général, ces compositions sont employées après un shampooing éventuellement précédé par l'un des traitements ci-dessus.

On a déjà utilisé dans ce but les céramides (EP 278505) ou les glycocéramides qui ont été associés à des esters de cholestérol dans le but de protéger la fibre capillaire. L'application sur cheveux de ces dernières compositions ou des céramides seuls conduit cependant à des performances cosmétiques insuffisantes, tant sur cheveux mouillés que sur cheveux séchés.

Les céramides sont généralement formulés dans des compositions épaisses (crème ou gel) contenant des épaississants dans le but d'améliorer la stabilité et la mise en suspension des céramiques dans les compositions aqueuses.

FR 2679770 décrit des compositions contenant un tensioactif cationique, un céramide et un alcool gras solide

WO 99/29293 décrit des crèmes à base de céramides comprenant un alcool gras liquide entre autres ingrédients.

Les produits liquides s'appliquent mieux sur les cheveux et se répartissent de façon homogène. Cependant, il est difficile d'obtenir des compositions liquides aqueuses stables contenant des composés insolubles dans l'eau tels que les composés de type céramide.

Or, la demanderesse a découvert de manière surprenante qu'en utilisant des compositions contenant au moins un tensioactif cationique, au moins un alcool gras liquide en association avec des composés de type céramide, on obtenait des compositions liquides stables et qui présentaient une amélioration importante des performances cosmétiques aussi bien sur cheveux mouillés que sur cheveux séchés.

En particulier, les propriétés cosmétiques telles que les propriétés d'assouplissement, de lissage des fibres sans alourdissement ou effet gras, de douceur et de brillance sont supérieures à celles d'une composition contenant un alcool gras solide généralement utilisé pour améliorer la stabilité des compositions. De plus, cette composition ne nécessite pas de temps de pause.

Cette découverte est à la base de la présente invention.

L'invention a donc pour objet une composition cosmétique liquide, destinée au traitement des matières kératiniques en particulier les cheveux, caractérisée par le fait qu'elle comprend dans un milieu aqueux cosmétiquement acceptable au moins un tensioactif cationique, au moins un alcool gras liquide et au moins un composé de type céramide.

L'invention a encore pour objet l'utilisation de la composition définie ci-dessus pour protéger les matières kératiniques, en particulier les cheveux, des agressions physiques ou chimiques.

Ces compositions permettent d'améliorer les propriétés cosmétiques en particulier la douceur et le lissage des cheveux.

Par composition liquide, on entend des compositions présentant une viscosité inférieure ou égale à 1000 cpoises (1 Pa.s) et de préférence comprise entre 10 et 500 cpoises (0,01 et 0,5 Pa.s) et plus particulièrement entre 10 et 100 cps (0,01 et 0,1 Pa.s)

La viscosité est mesurée à 25°C avec un viscosimètre Rheomat avec une aiguille No.1, à une vitesse de rotation de 200 tours/min, la mesure étant effectuée après 30 secondes de rotation (temps au bout duquel on observe une stabilisation de la viscosité et de la vitesse de rotation du mobile).

Selon la présente invention, on entend, par composé de type céramide, les céramides et/ou les glycocéramides et/ou les pseudocéramides et/ou les néocéramides, naturelles ou synthétiques.

Des composés de type céramides sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131.

Les composés de type céramide utilisables selon la présente invention répondent préférentiellement à la formule générale (I):

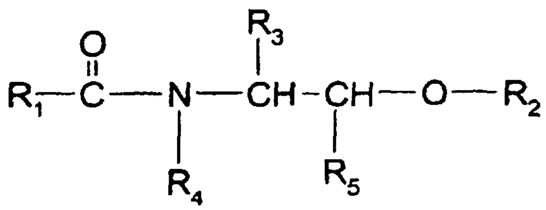

dans laquelle:

- $R_1$ désigne :
  - soit un radical hydrocarboné, linéaire ou ramifié, saturé ou insaturé, en $C_1$-$C_{50}$, de préférence en $C_5$-$C_{50}$, ce radical pouvant être substitué par un ou plusieurs groupements hydroxyle éventuellement estérifié par un acide $R_7COOH$, $R_7$ étant un radical hydrocarboné, saturé ou insaturé, linéaire ou ramifié, éventuellement mono ou polyhydroxylé, en $C_1$-$C_{35}$, le ou les hydroxyles du radical $R_7$ pouvant être estérifié par un acide gras saturé ou insaturé, linéaire ou ramifié, éventuellement mono ou polyhydroxylé, en $C_1$-$C_{35}$;
  - soit un radical R"-(NR-CO)-R', R désigne un atome d'hydrogène ou un radical hydrocarboné $C_1$-$C_{20}$ mono ou polyhydroxylé, préférentiellement monohydroxylé, R' et R" sont des radicaux hydrocarbonés dont la somme des atomes de carbone est comprise entre 9 et 30, R' étant un radical divalent.
  - soit un radical $R_8$-O-CO-$(CH2)_p$, $R_8$ désigne un radical hydrocarboné en $C_1$-$C_{20}$, p est un entier variant de 1 à 12.

- $R_2$ est choisi parmi un atome d'hydrogène, un radical de type saccharidique, en particulier un radical $(glycosyle)_n$, $(galactosyle)_m$ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;

- $R_3$ désigne un atome d'hydrogène ou un radical hydrocarboné en $C_1$-$C_{33}$, saturé ou insaturé, hydroxylé ou non, le ou les hydroxyles pouvant être estérifiés par un acide minéral ou un acide $R_7COOH$, $R_7$ ayant les mêmes significations que ci-dessus, le ou les hydroxyles pouvant être éthérifiés par un radical $(glycosyle)_n$, $(galactosyle)_m$, sulfogalactosyle, phosphoryléthylamine ou phosphoryléthylammonium, $R_3$ pouvant également être substitué par un ou plusieurs radicaux alkyle en $C_1$-$C_{14}$ ; de préférence, $R_3$ désigne un radical $\alpha$-hydroxyalkyle en $C_{15}$-$C_{26}$, le groupement hydroxyle étant éventuellement estérifié par un $\alpha$-hydroxyacide en $C_{16}$-$C_{30}$ ;

- $R_4$ désigne un atome d'hydrogène, un radical méthyle, éthyle, un radical hydrocarboné en $C_3$-$C_{50}$, saturé ou insaturé, linéaire ou ramifié, éventuellement hydroxylé ou un radical -$CH_2$-CHOH-$CH_2$-O-$R_6$ dans lequel $R_6$ désigne un radical hydrocarboné en $C_{10}$-$C_{26}$ ou un radical $R_8$-O-CO-$(CH2)_p$, $R_8$ désigne un radical hydrocarboné en $C_1$-$C_{20}$, p est un entier variant de 1 à 12,

- $R_5$ désigne un atome d'hydrogène ou un radical hydrocarboné en $C_1$-$C_{30}$ saturé ou insaturé, linéaire ou ramifié, éventuellement mono ou polyhydroxylé, le ou les hydroxyles pouvant être éthérifiés par un radical $(glycosyle)_n$, $(galactosyle)_m$, sulfogalactosyle, phosphoryléthylamine ou phosphoryléthylammonium,

sous réserve que lorsque $R_3$ et $R_5$ désignent hydrogène ou lorsque $R_3$ désigne hydrogène et $R_5$ désigne méthyle alors $R_4$ ne désigne pas un atome d'hydrogène, un radical méthyle ou éthyle.

Parmi les composés de formule (I), on préfère les céramides et/ou glycocéramides dont la structure est décrite par DOWNING dans Journal of Lipid Research Vol. 35, 2060-2068, 1994, ou ceux décrits dans la demande de brevet français FR-2 673 179, dont les enseignements sont ici inclus à titre de référence.

Les composés de type céramide plus particulièrement préférés selon l'invention sont les composés de formule (I) pour lesquels $R_1$ désigne un alkyle saturé ou insaturé dérivé d'acides gras en $C_{14}$-$C_{22}$ éventuellement hydroxylé; $R_2$ désigne un atome d'hydrogène ; et $R_3$ désigne un radical linéaire en $C_{11-17}$ éventuellement hydroxylé et de préférence en $C_{13-15}$.

De tels composés sont par exemple:

- le 2-N-linoléoylamino-octadécane-1,3-diol,
- le 2-N-oléoylamino-octadécane-1,3-diol,

- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1.3-diol,
- le 2-N-stéaroyl amino-octadécane-1,3,4 triol et en particulier la N-stéaroyl phytosphingosine,
- le 2-N-palmitoylamino-hexadécane-1,3-diol

ou les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (I) pour lesquels $R_1$ désigne un radical alkyle saturé ou insaturé dérivé d'acides gras en $C_{12}$-$C_{22}$ ; $R_2$ désigne un radical galactosyle ou sulfogalactosyle ; et $R_3$ désigne un radical hydrocarboné en $C_{12}$-$C_{22}$, saturé ou insaturé et de préférence un groupement $-CH=CH-(CH_2)_{12}-CH_3$.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (I) décrits dans les demandes de brevet EP-A-0227994, EP-A-0 647 617, EP-A-0 736 522 et WO 94/07844.

De tels composés sont par exemple le QUESTAMIDE H (bis-(N-hydroxyéthyl N-cétyl) malonamide) vendu par la société QUEST, le N-(2-hydroxyéthyl)-N-(3-cétyloxy-2-hydroxypropyl)amide d'acide cétylique .

On peut également utiliser le N-docosanoyl N-méthyl-D-glucamine décrit dans la demande de brevet WO94/24097.

La concentration en composés de type céramide peut varier entre 0,0001% et 20% en poids environ par rapport au poids total de la composition, et de préférence entre 0,001 et 10% environ et encore plus préférentiellement entre 0,005 et 3 % en poids.

Les alcools gras liquides à une température inférieure à 30°C sont choisis notamment parmi les alcools gras liquides en $C_{10}$-$C_{30}$, linéaires ou ramifiés, saturés ou insaturés ;

Plus particulièrement, les alcools gras liquides sont choisis parmi l'alcool laurique, l'alcool myristique, l'alcool isomyristique, l'alcool isostéarylique, l'alcool isocétylique, l'alcool isoarachidylique, l'octyl-2 dodécanol, le butyl-2 octanol et l'alcool oléique, et leurs mélanges.

De préférence, l'alcool gras est choisi parmi l'alcool isostéarylique et l'alcool isocétylique.

La concentration en alcools gras liquides selon l'invention peut varier entre 0,5% et 10% en poids environ par rapport au poids total de la composition, et de préférence entre 1 et 10% environ et encore plus préférentiellement entre 1,5 et 3 % en poids.

Les tensioactifs cationiques peuvent être choisis parmi :

A) les sels d'ammonium quaternaires de la formule générale (IV) suivante :

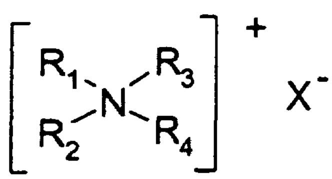

(IV)

dans laquelle X est un anion choisi dans le groupe des halogénures (chlorure, bromure ou iodure) ou alkyl($C_2$-$C_6$) sulfates plus particulièrement méthylsulfate, des phosphates, des alkyl-ou-alkylarylsulfonates, des anions dérivés d'acide organique tel que l'acétate ou le lactate.

, et

i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
R4 désigne un radical alkyle, linéaire ou ramifié, comportant de 20 à 30 atomes de carbone.
De préférence le tensioactif cationique est un sel (par exemple chlorure) de béhényl triméthyl ammonium.

ii) les radicaux R1 et R2, qui peuvent être identiques ou différents, représentent un radical aliphatique, linéaire ou

ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;

R3 et R4, identiques ou différents, désignent un radical alkyle, linéaire ou ramifié, comportant de 12 à 30 atomes de carbone, ledit radical comprenant au moins une fonction ester ou amide.

R3 et R4 sont notamment choisis parmi les radicaux alkyl($C_{12}$-$C_{22}$)amido alkyle($C_2$-$C_6$), alkyl($C_{12}$-$C_{22}$)acétate ;

De préférence le tensioactif cationique est un sel (par exemple chlorure) de stéaramidopropyl diméthyl (myristylacétate) ammonium.

B) - les sels d'ammonium quaternaire de l'imidazolinium, comme par exemple celui de formule (V) suivante :

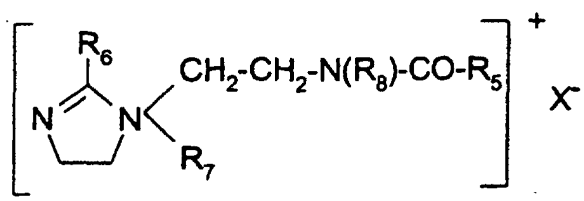

(V)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$ , $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple le Quaternium-27(CTFA 1997) ou le Quatemium-83 (CTFA 1997) commercialisés sous les dénominations "REWOQUAT" W 75, W90, W75PG, W75HPG par la société WITCO,

C) - les sels de diammonium quaternaire de formule (VI) :

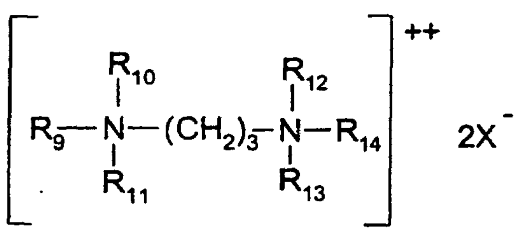

(VI)

dans laquelle $R_9$ désigne un radical aliphatique comportant environ de 16 à 30 atomes de carbone, $R_{10}$, $R_{11}$, $R_{12}$, $R_{13}$ et $R_{14}$, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

D) - les sels d'ammonium quaternaire contenant au moins une fonction ester de formule (VII) suivante :

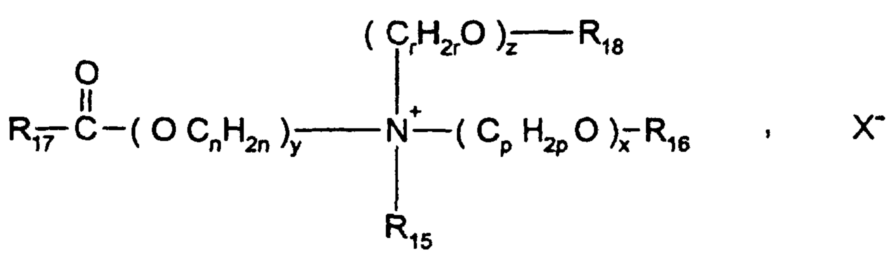

(VII)

dans laquelle:

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi parmi :
  - le radical

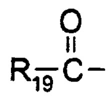

  - les radicaux $R_{20}$ hydrocarbonés en $C_1$-$C_{22}$ linéaires ou ramifiés, saturés ou insaturés,
  - l'atome d'hydrogène,
- $R_{18}$ est choisi parmi :
  - le radical

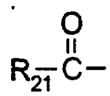

  - les radicaux $R_{22}$ hydrocarbonés en $C_1$-$C_6$ linéaires ou ramifiés, saturés ou insaturés,
  - l'atome d'hydrogène,
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.

On utilise plus particulièrement les sels d'ammonium de formule (VII) dans laquelle:

- $R_{15}$ désigne un radical méthyle ou éthyle,
- x et y sont égaux à 1 ;
- z est égal à 0 ou 1 ;
- n, p et r sont égaux à 2 ;
- $R_{16}$ est choisi parmi:
  - le radical

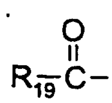

  - les radicaux méthyle, éthyle ou hydrocarbonés en $C_{14}$-$C_{22}$
  - l'atome d'hydrogène ;
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- $R_{18}$ est choisi parmi :

- le radical

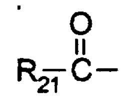

- l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Le tensioactif cationique est présent dans des concentrations allant de 0,2 à 10% en poids par rapport au poids total de la composition et de préférence de 0,5 à 5 % en poids et plus préférentiellement entre 1 et 3,5% en poids.

La composition de l'invention peut également contenir au moins un additif choisi parmi les épaississants, les parfums, les agents nacrants, les agents tensioactifs, les conservateurs, les filtres solaires, les silicones, les polymères anioniques ou non ioniques ou cationiques ou amphotères, les protéines, les hydrolysats de protéines, les acides gras, les alcools gras, les hydroxyacides, les vitamines, les provitamines telles que le panthénol, les huiles végétales, animales, minérales ou synthétiques et tout autre additif classiquement utilisé dans le domaine cosmétique qui n'affecte pas -les propriétés des compositions selon l'invention.

Ces additifs sont présents dans la composition selon l'invention dans des proportions pouvant aller de 0 à 50% en poids par rapport au poids total de la composition. La quantité précise de chaque additif est déterminée facilement par l'homme du métier selon sa nature et sa fonction.

Le milieu aqueux cosmétiquement acceptable peut être constitué uniquement par de l'eau ou par un mélange d'eau et d'au moins un solvant cosmétiquement acceptable tel les monoalcools, les polyalcools, les éthers de glycol et leur mélange. Les monoalcools sont notamment choisis parmi les alcools inférieurs en $C_1$-$C_4$, comme l'éthanol, l'isopropanol, le tertiobutanol, le n-butanol ; les alkylèneglycols comme le propylènegiycol, les éthers de glycols et leur mélange.

De préférence , la composition comprend de 50 à 95 % en poids d'eau par rapport au poids total de la composition.

Le pH des compositions est généralement compris entre 2 et 12 et de préférence entre 4 et 9. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent être plus particulièrement utilisées pour le traitement des matières kératiniques telles que les cheveux, la peau, les cils, les sourcils, les ongles, les lèvres, le cuir chevelu et plus particulièrement les cheveux.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin de la peau, des cheveux ou de toute autre matière kératinique.

Les compositions de l'invention peuvent également se présenter sous forme de shampooing, d'après-shampooing à rincer ou non, de compositions pour permanente, défrisage, coloration ou décoloration, ou encore sous forme de compositions à rincer, à appliquer avant ou après un shampooing, une coloration, une décoloration, une permanente ou un défrisage ou encore entre les deux étapes d'une permanente ou d'un défrisage.

Les compositions de l'invention sont utilisables plus spécialement comme composition capillaire dont l'application est suivie éventuellement d'un rinçage à l'eau.

Aussi, l'invention se rapporte à l'utilisation de cette composition comme ou pour la fabrication d'une composition à appliquer avant ou après tout traitement capillaire tel qu'un shampooing, une coloration ou une décoloration, une permanente ou un défrisage.

Les compositions selon l'invention peuvent être utilisées comme produits non-rincés notamment pour le maintien de la coiffure, la mise en forme ou le coiffage des cheveux.

Elles sont plus particulièrement des lotions de mise en plis, des lotions pour le brushing, des compositions de fixation (laques) et de coiffage.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.

Dans les exemples, MA signifie matière active.

Les compositions des exemples suivants sont par exemple obtenues en portant le mélange alcool gras, céramide à 70-80°C puis en ajoutant le mélange d'eau et de tensioactif cationique chauffé à la même température ; on agite ensuite vigoureusement avec une turbine pendant environ 10 minutes. On laisse ensuite refroidir sous agitation jusqu'à température ambiante.

**EXEMPLE 1**

On a préparé un après-shampooing rincé de composition suivante :

| | |
|---|---|
| Chlorure de béhényl triméthyl ammonium (GENAMIN KDM-F de HOECHST CHIMIE) | 2,4 gMA |
| Alcool isostéarylique | 2 g |
| N-oléoyldihydrosphingosine (céramide) | 0,4 g |
| Alcool cétylstéarylique (50/50 en poids) | 0,5 g |
| Silicone aminée en émulsion (DC949 de DOW CORNING) | 0,95 gMA |
| Parfum, conservateurs qs | |
| Eau déminéralisée qsp | 100 g |

La composition a une viscosité de 37 cps, elle est stable au moins 8 jours à 45°C. On applique cette composition sur des cheveux lavés et essorés. Les cheveux traités avec cette composition présentent d'excellentes propriétés cosmétiques de souplesse, de douceur, de toucher, de brillance sans alourdissement. Ces effets sont obtenus sans temps de pause.

## Revendications

1. Composition cosmétique liquide, **caractérisée par le fait qu'**elle comprend dans un milieu aqueux cosmétiquement acceptable au moins un alcool gras liquide, au moins un composé de type céramide et au moins un tensioactif cationique.

2. Composition selon la revendication 1, **caractérisée par le fait que** le composé de type céramide répond à la formule générale (I):

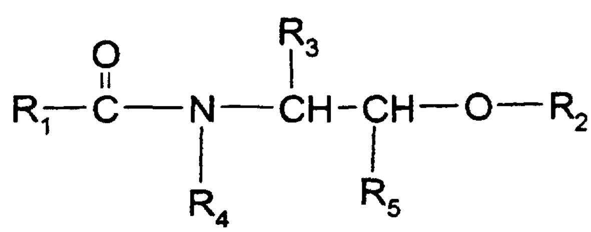

dans laquelle :

- $R_1$ désigne :

- soit un radical hydrocarboné, linéaire ou ramifié, saturé ou insaturé, en $C_1$-$C_{50}$, de préférence en $C_5$-$C_{50}$, ce radical pouvant être substitué par un ou plusieurs groupements hydroxyle éventuellement estérifié par un acide $R_7COOH$, $R_7$ étant un radical hydrocarboné, saturé ou insaturé, linéaire ou ramifié, éventuellement mono ou polyhydroxylé, en $C_1$-$C_{35}$, le ou les hydroxyles du radical $R_7$ pouvant être estérifié par un acide gras saturé ou insaturé, linéaire ou ramifié, éventuellement mono

ou polyhydroxylé, en $C_1$-$C_{35}$;

- soit un radical R"-(NR-CO)-R', R désigne un atome d'hydrogène ou un radical hydrocarboné $C_1$-$C_{20}$ mono ou polyhydroxylé, préférentiellement monohydroxylé, R' et R" sont des radicaux hydrocarbonés dont la somme des atomes de carbone est comprise entre 9 et 30, R' étant un radical divalent.
- soit un radical $R_8$-O-CO-$(CH2)_p$, $R_8$ désigne un radical hydrocarboné en $C_1$-$C_{20}$, p est un entier variant de 1 à 12.

- $R_2$ est choisi parmi un atome d'hydrogène, un radical de type saccharidique, en particulier un radical $(glycosyle)_n$, $(galactosyle)_m$ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;

- $R_3$ désigne un atome d'hydrogène ou un radical hydrocarboné en $C_1$-$C_{33}$, saturé ou insaturé, hydroxylé ou non, le ou les hydroxyles pouvant être estérifiés par un acide minéral ou un acide $R_7COOH$, $R_7$ ayant les mêmes significations que ci-dessus, le ou les hydroxyles pouvant être éthérifiés par un radical $(glycosyle)_n$, $(galactosyle)_m$, sulfogalactosyle, phosphoryléthylamine ou phosphoryléthylammonium, $R_3$ pouvant également être substitué par un ou plusieurs radicaux alkyle en $C_1$-$C_{14}$ ; de préférence, $R_3$ désigne un radical $\alpha$-hydroxyalkyle en $C_{15}$-$C_{26}$, le groupement hydroxyle étant éventuellement estérifié par un $\alpha$-hydroxyacide en $C_{16}$-$C_{30}$ ;

- $R_4$ désigne un atome d'hydrogène, un radical méthyle, éthyle, un radical hydrocarboné en $C_3$-$C_{50}$, saturé ou insaturé, linéaire ou ramifié, éventuellement hydroxylé ou un radical -$CH_2$-CHOH-$CH_2$-O-$R_6$ dans lequel $R_6$ désigne un radical hydrocarboné en $C_{10}$-$C_{26}$ ou un radical $R_8$-O-CO-$(CH2)_p$, $R_8$ désigne un radical hydrocarboné en $C_1$-$C_{20}$, p est un entier variant de 1 à 12,

- $R_5$ désigne un atome d'hydrogène ou un radical hydrocarboné en $C_1$-$C_{30}$ saturé ou insaturé, linéaire ou ramifié, éventuellement mono ou polyhydroxylé, le ou les hydroxyles pouvant être éthérifiés par un radical $(glycosyle)_n$, $(galactosyle)_m$, sulfogalactosyle, phosphoryléthylamine ou phosphoryléthylammonium,

sous réserve que lorsque $R_3$ et $R_5$ désignent hydrogène ou lorsque $R_3$ désigne hydrogène et $R_5$ désigne méthyle alors $R_4$ ne désigne pas un atome d'hydrogène, un radical méthyle ou éthyle.

3. Composition selon l'une quelconque des revendications précédentes, **caractérisé en ce que** le composé de type céramide est choisi dans le groupe constitué par :

- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol,
- le 2-N-palmitoylamino-hexadécane-1,3-diol,

ou les mélanges de ces composés.

4. Composition selon l'une quelconque des revendications 1 ou 2, **caractérisé en ce que** le composé de type céramide est choisi parmi le bis-(N-hydroxyéthyl N-cétyl) malonamide, le N-(2-hydroxyéthyl)-N-(3-cétyloxy-2-hydroxypropyl)amide d'acide cétylique) et le N-docosanoyl N-méthyl-D-glucamine.

5. Composition selon l'une quelconque des revendications précédentes, **caractérisée par le fait que** le ou les composés de type céramide sont présents dans des concentrations allant de 0,0001 à 20% en poids par rapport au poids total de la composition et de préférence de 0,001 à 10 % en poids et plus préférentiellement entre 0,005 et 3% en poids.

**6.** Composition selon l'une quelconque des revendications précédentes, **caractérisée par le fait que** les alcools gras liquides sont choisis parmi l'alcool laurique, l'alcool myristique, l'alcool isomyristique, l'alcool isostéarylique, l'alcool isocétylique, l'alcool isoarachidylique, l'octyl-2 dodécanol, le butyl-2 octanol et l'alcool oléique, et leurs mélanges.

**7.** Composition selon la revendication précédente, **caractérisée par le fait que** le l'alcool gras est choisi parmi l'alcool isostéarylique et l'alcool isocétylique.

**8.** Composition selon l'une quelconque des revendications précédentes, **caractérisée par le fait que** la concentration en alcools gras liquides varie entre 0,5% et 10% en poids environ par rapport au poids total de la composition, et de préférence entre 1 et 10% environ et encore plus préférentiellement entre 1,5 et 3 % en poids.

**9.** Composition selon l'une quelconque des revendications précédentes, **caractérisée par le fait que** le dit tensioactif cationique est choisi parmi :

A) les sels d'ammonium quaternaires de la formule générale (IV) suivante :

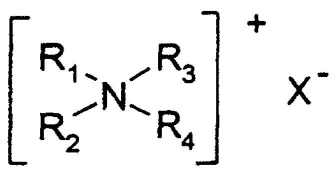

(IV)

dans laquelle X est un anion choisi dans le groupe des halogénures (chlorure, bromure ou iodure) ou alkyl($C_2$-$C_6$)sulfates plus particulièrement méthylsulfate, des phosphates, des alkyl-ou-alkylarylsulfonates, des anions dérivés d'acide organique tel que l'acétate ou le lactate.
, et

i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes,
R4 désigne un radical alkyle, linéaire ou ramifié, comportant de 20 à 30 atomes de carbone.
ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
R3 et R4, identiques ou différents, désignent un radical alkyle, linéaire ou ramifié, comportant de 12 à 30 atomes de carbone, ledit radical comprenant au moins une fonction ester ou amide.
R3 et R4 sont notamment choisis parmi les radicaux alkyl($C_{12}$-$C_{22}$)amido alkyle($C_2$-$C_6$), alkyl($C_{12}$-$C_{22}$) acétate ;

B) - les sels d'ammonium quaternaire de l'imidazolinium, comme par exemple celui de formule (V) suivante :

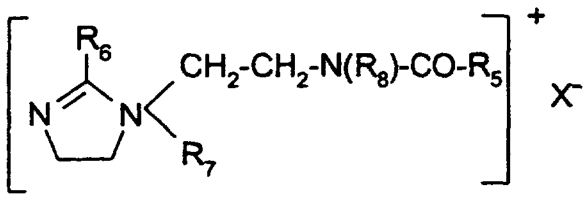

(V)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$ , $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates.

C) - les sels de diammonium quaternaire de formule (VI):

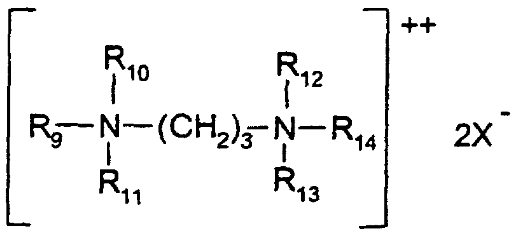

(VI)

dans laquelle $R_9$ désigne un radical aliphatique comportant environ de 16 à 30 atomes de carbone, $R_{10}$, $R_{11}$, $R_{12}$, $R_{13}$ et $R_{14}$ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

D) - les sels d'ammonium quaternaire contenant au moins une fonction ester de formule (VII) suivante :

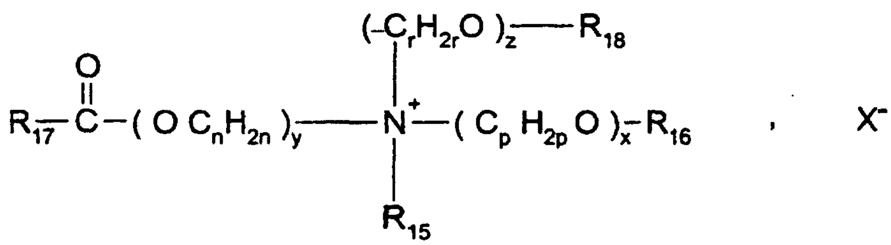

(VII)

dans laquelle:

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi Parmi :

  - le radical

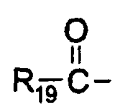

  - les radicaux $R_{20}$ hydrocarbonés en $C_1$-$C_{22}$ linéaires ou ramifiés, saturés ou insaturés,
  - l'atome d'hydrogène,

- $R_{18}$ est choisi parmi

  - le radical

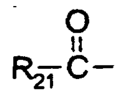

  - les radicaux $R_{22}$ hydrocarbonés en $C_1$-$C_6$ linéaires ou ramifiés, saturés ou insaturés,
  - l'atome d'hydrogène,
  - $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
  - n, p et r, identiques ou différents, sont des entiers valant de 2 à 6 ;
  - y est un entier valant de 1 à 10 ;
  - x et z, identiques ou différents, sont des entiers valant de 0 à 10 ;
  - $X^-$ est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.

**10.** Composition selon l'une quelconque des revendications précédentes, **caractérisée par le fait que** ledit tensioactif cationique est choisi parmi les sels de béhényl triméthyl ammonium, les sels de stéaramidopropyl diméthyl (myristylacétate) ammonium, le Quaternium-27 ou le Quaternium-83.

**11.** Composition selon l'une quelconque des revendications précédentes, **caractérisée par le fait que** ledit tensioactif cationique est présent dans des concentrations allant de 0,2 à 10% en poids par rapport au poids total de la composition et de préférence de 0,5 à 5 % en poids et plus préférentiellement entre 1 et 3.5% en poids.

**12.** Composition selon l'une quelconque des revendications précédentes, **caractérisée par le fait que** le milieu cosmétiquement acceptable est constitué par de l'eau ou un mélange d'eau et d'au moins un solvant cosmétiquement acceptable.

**13.** Composition selon la revendication 12, **caractérisée par le fait que** les solvants cosmétiquement acceptables sont choisis dans le groupe constitué par les monoalcools, les polyalcools. les éthers de glycol, et leurs mélanges.

**14.** Composition selon l'une quelconque des revendications 1 à 13, **caractérisée par le fait qu'**elle comprend en outre au moins un additif choisi parmi les épaississants, les parfums, les agents nacrants, les conservateurs, les filtres solaires, les polymères anioniques ou non ioniques ou cationiques ou amphotères, les protéines, les hydrolysats de protéines, les acides gras à chaînes linéaires ou ramifiées en $C_{16}$-$C_{40}$ tels que l'acide méthyl-18 eicosanoique, les hydroxyacides, les vitamines, le panthénol, et les esters gras.

**15.** Composition selon l'une quelconque des revendications 1 à 14, **caractérisée par le fait qu'**elle se présente sous forme de shampooing, de composition pour la permanente, le défrisage, la coloration ou la décoloration des cheveux, de composition lavantes pour la peau.

**16.** Utilisation d'une composition telle que définie dans l'une quelconque des revendications 1 à 15 comme ou pour la fabrication d'une composition à appliquer avant ou après tout traitement capillaire tel qu'un shampooing, une coloration ou une décoloration, une permanente ou un défrisage, d'une composition à rincer à appliquer entre les deux étapes d'une permanente ou d'un défrisage, d'après shampooing.

**17.** Procédé de traitement des matières kératiniques, telles que les cheveux, **caractérisé en ce qu'**il consiste à appliquer sur lesdites matières une composition cosmétique selon l'une des revendications 1 à 15, puis à effectuer éventuellement un rinçage à l'eau.

**18.** Utilisation d'une composition telle que définie selon l'une quelconque des revendications 1 à 15 pour protéger les matières kératiniques, en particulier les cheveux, des agressions physiques ou chimiques.

## Patentansprüche

1. Flüssige kosmetische Zusammensetzung, **dadurch gekennzeichnet, dass** sie in einem kosmetisch akzeptablen wässrigen Medium mindestens einen flüssigen Fettalkohol, mindestens eine Verbindung vom Ceramidtyp und mindestens einen kationischen grenzflächenaktiven Stoff enthält.

2. Zusammensetzung nach Anspruch 1, **dadurch gekennzeichnet, dass** die Verbindung vom Ceramidtyp der folgenden allgemeinen Formel (I) entspricht:

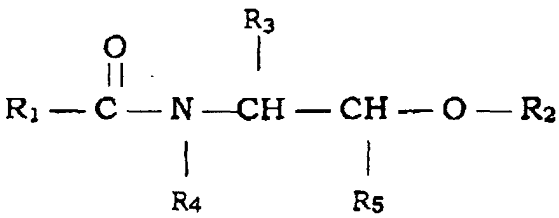

worin bedeuten:

- Ri:

  - entweder eine geradkettige oder verzweigte, gesättigte oder ungesättigte Kohlenwasserstoffgruppe mit 1 bis 50 und vorzugsweise 5 bis 50 Kohlenstoffatomen, wobei diese Gruppe mit einer oder mehreren Hydroxygruppen substituiert sein kann, die gegebenenfalls mit einer Säure $R_7COOH$ verestert sind, wobei $R_7$ eine gesättigte oder ungesättigte, geradkettige oder verzweigte, gegebenenfalls ein- oder mehrfach hydroxylierte Kohlenwasserstoffgruppe mit 1 bis 35 Kohlenstoffatomen ist, wobei die Hydroxygruppe(n) der Gruppe $R_7$ mit einer gesättigten oder ungesättigten, geradkettigen oder verzweigten, gegebenenfalls ein- oder mehrfach hydroxylierten $C_{1-35}$-Fettsäure verestert sein können;
  - oder eine Gruppe R''-(NR-CO)-R', wobei R ein Wasserstoffatom oder eine ein- oder mehrfach hydroxylierte $C_{1-20}$-Kohlenwasserstoffgruppe bedeutet, wobei R vorzugsweise monohydroxyliert ist, und die Gruppen R' und R'' Kohlenwasserstoffgruppen sind, deren Summe der Kohlenstoffatome im Bereich von 9 bis 30 liegt, wobei R' eine zweiwertige Gruppe ist;
  - oder eine Gruppe $R_8$-O-CO-$(CH_2)_p$, wobei $R_8$ eine $C_{1-20}$-Kohlenwasserstoffgruppe ist und p eine ganze Zahl im Bereich von 1 bis 12 bedeutet;

- $R_2$ ist unter einem Wasserstoffatom, einer Gruppe vom Saccharidtyp und insbesondere (Glykosyl)n, (Galactosyl)m oder Sulfogalactosyl, Sulfat oder Phosphat, Phosphorylethylamin und Phosphorylethylammonium ausgewählt, wobei n eine ganze Zahl von 1 bis 4 und m eine ganze Zahl von 1 bis 8 bedeutet;
- $R_3$ ein Wasserstoffatom oder eine gesättigte oder ungesättigte, hydroxylierte oder nicht hydroxylierte $C_{1-33}$-Kohlenwasserstoffgruppe, wobei die Hydroxygruppe(n) mit einer anorganischen Säure oder einer Säure $R_7COOH$ verestert sein können, wobei $R_7$ die oben angegebenen Bedeutungen aufweist, wobei die Hydroxygruppe(n) mit einer Gruppe $(Glykosyl)_n$, $(Galactosyl)_m$, Sulfogalactosyl, Phosphorylethylamin oder Phosphorylethylammonium verethert sein können und wobei $R_3$ auch mit einer oder mehreren $C_{1-14}$-Alkylgruppen substituiert sein kann; die Gruppe $R_3$ bedeutet vorzugsweise eine $\alpha$-Hydroxyalkylgruppe mit 15 bis 26 Kohlenstoffatomen, wobei die Hydroxygruppe gegebenenfalls mit einer $\alpha$-Hydroxysäure mit 16 bis 30 Kohlenstoffatomen verestert ist;
- $R_4$ ein Wasserstoffatom, Methyl, Ethyl, eine gesättigte oder ungesättigte, geradkettige oder verzweigte, gegebenenfalls hydroxylierte $C_{3-50}$-Kohlenwasserstoffgruppe oder eine Gruppe -$CH_2$-CHOH-$CH_2$-O-$R_6$, wobei $R_6$ eine $C_{10-26}$-Kohlenwasserstoffgruppe oder eine Gruppe $R_8$-O-CO-$(CH_2)_p$ ist, wobei $R_8$ eine $C_{1-20}$-Kohlenwasserstoffgruppe bedeutet und p eine ganze Zahl im Bereich von 1 bis 12 ist;
- $R_5$ ein Wasserstoffatom oder eine gesättigte oder ungesättigte, geradkettige oder verzweigte, gegebenenfalls ein- oder mehrfach hydroxylierte $C_{1-30}$-Kohlenwasserstoffgruppe, wobei die Hydroxygruppe(n) mit einer Gruppe $(Glykosyl)_n$, $(Galactosyl)_m$, Sulfogalactosyl, Phosphorylethylamin oder Phosphorylethylammonium verethert sein können,

mit der Maßgabe, dass $R_4$ nicht Wasserstoff, Methyl oder Ethyl bedeutet, wenn $R_3$ und $R_5$ Wasserstoff bedeuten oder wenn $R_3$ Wasserstoff und $R_5$ Methyl bedeutet.

**3.** Zusammensetzung nach einem der vorhergehenden Ansprüche, **dadurch gekennzeichnet, dass** die Verbindung vom Ceramidtyp unter den folgenden Verbindungen ausgewählt ist:

- 2-N-Linoleoylamin-octadecan-1,3-diol,
- 2'-N-Oleoylamin-octadecan- 1,3-diol,
- 2-N-Palinitoylamino-octadecan-1,3-diol,
- 2-N-Stearoylamino-octadecan-1,3-diol,
- 2-N-Behenoylamino-octadecan-1,3-diol,
- 2-N-[2-Hydroxy-palmitoyl]-amino-octadecan-1,3-diol,
- 2-N-Stearoylamino-octadecan-1,3,4-triol,
- 2-N-Palmitoylamino-hexadecan-1,3-diol

oder den Gemischen dieser Verbindungen.

**4.** Zusammensetzung nach einem der Ansprüche 1 oder 2, **dadurch gekennzeichnet, dass** die Verbindung vom Ceramidtyp unter Bis(N-hydroxyethyl-N-cetyl)malonamid, dem N-(2-Hydroxyethyl)-N-(3-cetyloxy-2-hydroxypropyl)amid von Cetylsäure und N-Docosanoyl-N-methyl-D-glucamin ausgewählt ist.

**5.** Zusammensetzung nach einem der vorhergehenden Ansprüche, **dadurch gekennzeichnet, dass** die Verbindung (en) vom Ceramidtyp in Konzentrationen von 0,0001 bis 20 Gew.-%, bezogen auf das Gesamtgewicht der Zusammensetzung, vorzugsweise 0,001 bis 10 Gew.-% und noch bevorzugter 0,005 bis 3 Gew.-% enthalten sind.

**6.** Zusammensetzung nach einem der vorhergehenden Ansprüche, **dadurch gekennzeichnet, dass** die flüssigen Fettalkohole unter Laurylalkohol, Myristylalkohol, Isomyristylalkohol, Isostearylalkohol, Isocetylalkohol, Isoarachidylalkohol, 2-Octyldodecanol, 2-Butyloctanol und Oleylalkohol und deren Gemischen ausgewählt sind.

**7.** Zusammensetzung nach einem der vorhergehenden Ansprüche, **dadurch gekennzeichnet, dass** der Fettalkohol unter Isostearylalkohol und Isocetylalkohol ausgewählt ist.

**8.** Zusammensetzung nach einem der vorhergehenden Ansprüche, **dadurch gekennzeichnet, dass** die Konzentration der flüssigen Fettalkohole im Bereich von etwa 0,5 bis 10 Gew.-%, bezogen auf das Gesamtgewicht der Zusammensetzung, vorzugsweise etwa 1 bis 10 Gew.-% und noch bevorzugter 1,5 bis 3 Gew.-% liegt.

**9.** Zusammensetzung nach einem der vorhergehenden Ansprüche, **dadurch gekennzeichnet, dass** der kationische grenzflächenaktive Stoff ausgewählt ist unter:

A) den quartären Ammoniumsalzen der folgenden allgemeinen Formel (IV):

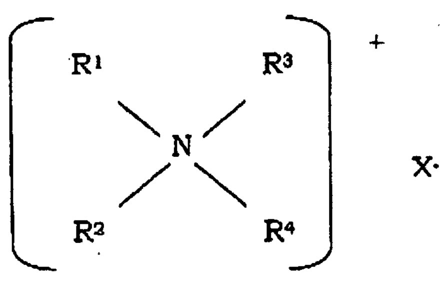

(IV)

worin X ein Anion bedeutet, das unter den Halogeniden (Chlorid, Bromid oder Iodid) oder $C_{2-6}$-Alkylsulfaten und insbesondere Methylsulfat, Phosphaten, Alkyl- oder Alkylarylsulfonaten und von organischen Säuren abgeleiteten Anionen, wie Acetat oder Lactat, ausgewählt ist; und

i) die Gruppen $R_1$ bis $R_3$, die gleich oder verschieden sein können, bedeuten eine geradkettige oder verzweigte aliphatische Gruppe mit 1 bis 4 Kohlenstoffatomen oder eine aromatische Gruppe, wie Aryl oder

Alkylaryl. Die aliphatischen Gruppen können Heteroatome enthalten, wie beispielsweise insbesondere Sauerstoff, Stickstoff, Schwefel und Halogene; $R_4$ bedeutet eine geradkettige oder verzweigte Alkylgruppe mit 20 bis 30 Kohlenstoffatomen;

ii) die Gruppen $R_1$ und $R_2$, die gleich oder verschieden sein können, bedeuten eine geradkettige oder verzweigte aliphatische Gruppe mit 1 bis 4 Kohlenstoffatomen oder eine aromatische Gruppe, wie Aryl oder Alkylaryl. Die aliphatischen Gruppen können Heteroatome enthalten, wie insbesondere Sauerstoff, Stickstoff, Schwefel und Halogene. Die aliphatischen Gruppen sind beispielsweise unter den Gruppen Alkyl, Alkoxy, Alkylamid und Hydroxyalkyl ausgewählt, die etwa 1 bis 4 Kohlenstoffatome enthalten; die Gruppen $R_3$ und $R_4$, die gleich oder verschieden sind, bedeuten eine geradkettige oder verzweigte Alkylgruppe mit 12 bis 30 Kohlenstoffatomen, wobei die Gruppe mindestens eine Ester- oder Amidfunktion enthält.

Die Gruppen $R_3$ und $R_4$ sind insbesondere unter den Gruppen $C_{12-22}$-Alkylamido-$C_{2-6}$-alkyl oder $C_{12-22}$-Alkylacetat ausgewählt.

B) den quartären Imidazoliniumsalzen, beispielsweise den Salzen der folgenden Formel (V):

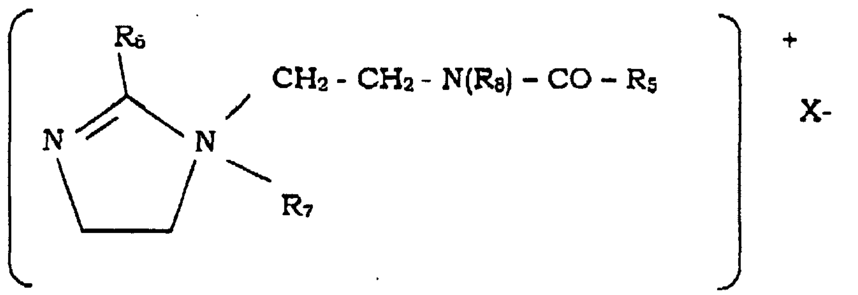

(V)

worin $R_5$ eine Alkenyl- oder Alkylgruppe mit 8 bis 30 Kohlenstoffatomen bedeutet, die beispielsweise von Talgfettsäuren abgeleitet sind, $R_6$ ein Wasserstoffatom, eine $C_{1-4}$-Alkylgruppe oder eine Alkenyl- oder Alkylgruppe mit 8 bis 30 Kohlenstoffatomen bedeutet, $R_7$ eine $C_{1-4}$-Alkylgruppe bedeutet, $R_8$ ein Wasserstoffatom oder eine $C_{1-4}$-Alkylgruppe bedeutet und X ein Anion ist, das unter den Halogeniden, Phosphaten, Acetaten, Lactaten, Alkylsulfaten und Alkyloder Alkylarylsulfonaten ausgewählt ist.

C) den quartären Diammoniumsalzen der Formel (VI):

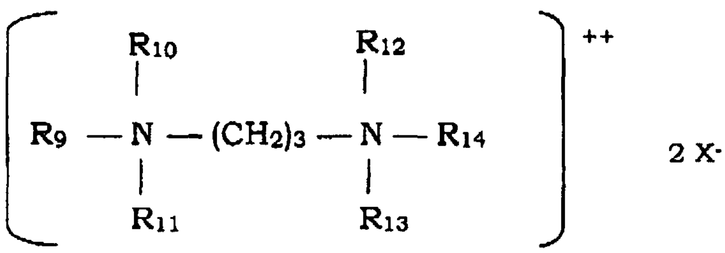

(VI)

worin $R_9$ eine aliphatische Gruppe mit etwa 16 bis 30 Kohlenstoffatomen bedeutet, $R_{10}$, $R_{11}$, $R_{12}$, $R_{13}$ und $R_{14}$, die gleich oder verschieden sind, unter Wasserstoff oder einer, Alkylgruppe mit 1 bis 4 Kohlenstoffatomen ausgewählt sind, und X ein Anion ist, das unter den Halogeniden, Acetaten, Phosphaten, Nitraten und Methylsulfaten ausgewählt ist.

Diese quartären Diammoniumsalze umfassen insbesondere das Propantalgdiammoniumdichlorid;

D) quartären Ammoniumsalzen, die mindestens eine Esterfunktion enthalten, der folgenden Formel (VII):

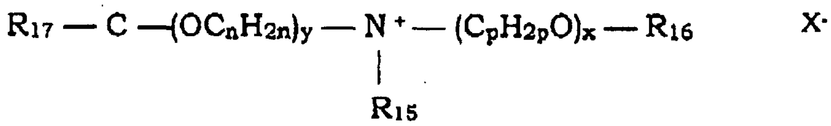

(VII)

wobei in der Formel:

wobei in der Formel:

- $R_{15}$ unter den $C_{1-6}$-Alkylgruppen und den Hydroxyalkyloder Dihydroxyalkylgruppen mit 1 bis 6 Kohlenstoffatomen ausgewählt ist;
- $R_{16}$ ausgewählt ist unter:
  - der Gruppe

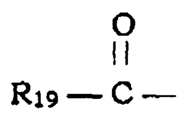

  - den gesättigten oder ungesättigten, geradkettigen oder verzweigten Gruppen $R_{20}$ auf Kohlenwasserstoffbasis mit 1 bis 22 Kohlenstoffatomen,
  - dem Wasserstoffatom,
- $R_{18}$ ist ausgewählt unter:
  - der Gruppe

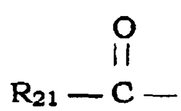

  - den gesättigten oder ungesättigten, geradkettigen oder verzweigten Gruppen $R_{22}$ auf Kohlenwasserstoffbasis mit 1 bis 6 Kohlenstoffatomen,
  - dem Wasserstoffatom,
- $R_{17}$, $R_{19}$ und $R_{21}$, die gleich oder verschieden sind, sind unter den geradkettigen oder verzweigten, gesättigten oder ungesättigten Kohlenwasserstoffgruppen mit 7 bis 21 Kohlenstoffatomen ausgewählt;
- n, p und r, die gleich oder verschieden sind, sind ganze Zahlen von 2 bis 6;
- y ist eine ganze Zahl von 1 bis 10;
- x und z, die gleich oder verschieden sind, bedeuten 0 oder ganze Zahlen von 1 bis 10; und
- $X^-$ ist ein einfaches oder komplexes, organisches oder anorganisches Anion,

mit der Maßgabe, dass, wenn die Summe x + y + z im Bereich von 1 bis 15 liegt, $R_{16}$ $R_{20}$ bedeutet, wenn x 0 ist, und $R_{18}$ $R_{22}$ bedeutet, wenn z 0 ist.

10. Zusammensetzung nach einem der vorhergehenden Ansprüche, **dadurch gekennzeichnet, dass** der kationische grenzflächenaktive Stoff unter Behenyltrimethylammoniumsalzen, Stearamidopropyldimethyl(myristylacetat)ammoniumsalzen, Quaternium-27 oder Quaternium-83 ausgewählt ist.

11. Zusammensetzung nach einem der vorhergehenden Ansprüche, **dadurch gekennzeichnet, dass** der kationische grenzflächenaktive Stoff in Konzentrationen von 0,2 bis 10 Gew.-%, bezogen auf das Gesamtgewicht der Zusammensetzung, vorzugsweise 0,5 bis 5 Gew.-% und noch bevorzugter 1 bis 3,5 Gew.-% enthalten ist.

**12.** Zusammensetzung nach einem der vorhergehenden Ansprüche, **dadurch gekennzeichnet, dass** das kosmetisch akzeptable Medium aus Wasser oder einem Gemisch von Wasser und mindestens einem kosmetisch akzeptablen Lösungsmittel besteht.

**13.** Zusammensetzung nach Anspruch 12, **dadurch gekennzeichnet, dass** die kosmetisch akzeptablen Lösungsmittel unter den Monoalkoholen, Polyalkoholen, Glykolethern und deren Gemischen ausgewählt ist.

**14.** Zusammensetzung nach einem der Ansprüche 1 bis 13, **dadurch gekennzeichnet, dass** sie ferner mindestens einen Zusatzstoff enthält, der unter den Verdickungsmitteln, Parfums, Perlglanzpigmenten, Konservierungsmitteln, Sonnenschutzfiltern, anionischen, nichtionischen, kationischen oder amphoteren Polymeren, Proteinen, Proteinhydrolysaten, geradkettigen oder verzweigten Fettsäuren mit 16 bis 40 Kohlenstoffatomen, wie 8-Methyleicosansäure, Hydroxysäuren, Vitaminen, Panthenol und Fettsäureestern ausgewählt ist.

**15.** Zusammensetzung nach einem der Ansprüche 1 bis 14, **dadurch gekennzeichnet, dass** sie als Haarwaschmittel, Zusammensetzung für permanente Verformungen, Entkräuselungen, Färbungen oder Entfärbungen der Haare oder reinigende Zusammensetzung für die Haut vorliegt.

**16.** Verwendung nach einem der Ansprüche 1 bis 15 als Zusammensetzung, die vor oder nach einer beliebigen Haarbehandlung aufgetragen wird, beispielsweise einer Haarwäsche, einer Färbung oder Entfärbung, permanenten Verformung oder Entkräuselung, als Zusammensetzung, die ausgespült wird und zwischen den beiden Schritten einer permanenten Verformung oder Entkräuselung aufgetragen wird, oder als Konditioner oder zur Herstellung solcher Zusammensetzungen.

**17.** Verfahren zur Behandlung von Keratinsubstanzen, wie den Haaren, **dadurch gekennzeichnet, dass** es darin besteht, auf die Keratinsubstanzen eine kosmetische Zusammensetzung nach einem der Ansprüche 1 bis 15 aufzutragen und gegebenenfalls mit Wasser zu spülen.

**18.** Verwendung einer Zusammensetzung nach einem der Ansprüche 1 bis 15 zum Schutz der Keratinsubstanzen und insbesondere zum Schutz der Haare gegen physikalische oder chemische Angriffe.

## Claims

**1.** Liquid cosmetic composition, **characterized in that** it comprises, in a cosmetically acceptable aqueous medium, at least one liquid fatty alcohol, at least one ceramide-type compound and at least one cationic surfactant.

**2.** Composition according to Claim 1, **characterized in that** the ceramide-type compound corresponds to the general formula (I):

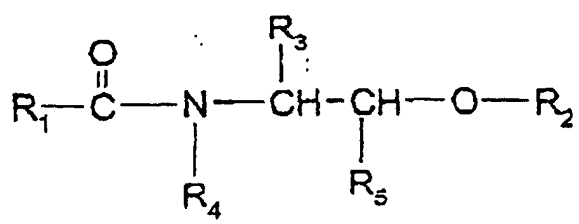

in which:

- $R_1$ denotes:

- either a saturated or unsaturated, linear or branched, $C_1$-$C_{50}$, preferably $C_5$-$C_{50}$, hydrocarbon radical, it being possible for this radical to be substituted with one or more hydroxyl groups optionally esterified by an acid $R_7COOH$, $R_7$ being an optionally mono- or polyhydroxylated, linear or branched, saturated or unsaturated, $C_1$-$C_{35}$ hydrocarbon radical, it being possible for the hydroxyl(s) of the $R_7$ radical to be esterified by an optionally

mono- or polyhydroxylated, linear or branched, saturated or unsaturated, $C_1$-$C_{35}$ fatty acid;

- or a radical R''-(NR-CO)-R', R denotes a hydrogen atom or a mono- or polyhydroxylated, preferably monohydroxylated, $C_1$-$C_{20}$ hydrocarbon radical, R' and R'' are hydrocarbon radicals of which the sum of the carbon atoms is between 9 and 30, R' being a divalent radical;
- or a radical $R_8$-O-CO- $(CH_2)_p$, $R_8$ denotes a $C_1$-$C_{20}$ hydrocarbon radical, p is an integer varying from 1 to 12;
- $R_2$ is chosen from a hydrogen atom, a saccharide-type radical, in particular a $(glycosyl)_n$, $(galactosyl)_m$ or sulphogalactosyl radical, a sulphate or phosphate residue, a phosphorylethylamine radical and a phosphorylethylammonium radical, in which n is an integer varying from 1 to 4 and m is an integer varying from 1 to 8;
- $R_3$ denotes a hydrogen atom or a hydroxylated or nonhydroxylated, saturated or unsaturated, $C_1$-$C_{33}$ hydrocarbon radical, it being possible for the hydroxyl(s) to be esterified by an inorganic acid or an acid $R_7COOH$, $R_7$ having the same meanings as above, it being possible for the hydroxyl(s) to be etherified by a $(glycosyl)_n$, $(galactosyl)_m$, sulphogalactosyl, phosphorylethylamine or phosphorylethylammonium radical, it being also possible for $R_3$ to be substituted with one or more $C_1$-$C_{14}$ alkyl radicals; preferably, $R_3$ denotes a $C_{15}$-$C_{26}$ $\alpha$-hydroxyalkyl radical, the hydroxyl group being optionally esterified by a $C_{16}$-$C_{30}$ $\alpha$-hydroxy acid;
- $R_4$ denotes a hydrogen atom, a methyl or ethyl radical, an optionally hydroxylated, linear or branched, saturated or unsaturated, $C_3$-$C_{50}$ hydrocarbon radical, a radical $-CH_2-CHOH-CH_2-O-R_6$

in which $R_6$ denotes a $C_{10}$-$C_{26}$ hydrocarbon radical or a radical $R_8$-O-CO-$(CH_2)_p$, $R_8$ denotes a $C_1$-$C_{20}$ hydrocarbon radical, p is an integer varying from 1 to 12,

- $R_5$ denotes a hydrogen atom or an optionally mono- or polyhydroxylated, linear or branched, saturated or unsaturated, $C_1$-$C_{30}$ hydrocarbon radical, it being possible for the hydroxyl(s) to be etherified by a $(glycosyl)_n$, $(galactosyl)_m$, sulphogalactosyl, phosphorylethylamine or phosphorylethylammonium radical;

with the proviso that when $R_3$ and $R_5$ denote hydrogen or when $R_3$ denotes hydrogen and $R_5$ denotes methyl, then $R_4$ does not denote a hydrogen atom, or a methyl or ethyl radical.

**3.** Composition according to any one of the preceding claims, **characterized in that** the ceramide-type compound is chosen from the group consisting of:

- 2-(N-linoleoylamino)-1,3-octadecanediol,
- 2-(N-oleoylamino)-1,3-octadecanediol,
- 2-(N-palmitoylamino)-1,3-octadecanediol,
- 2-(N-stearoylamino)-1,3-octadecanediol,
- 2-(N-behenoylamino)-1,3-octadecanediol,
- 2-[N-(2-hydroxypalmitoyl)amino]-1,3-octadecanediol,
- 2-(N-stearoylamino)-1,3,4-octadecanetriol,
- 2-(N-palmitoylamino)-1,3-hexadecanediol,

or mixtures of these compounds.

**4.** Composition according to either of Claims 1 and 2, **characterized in that** the ceramide-type compound is chosen from bis(N-hydroxyethyl-N-cetyl)malonamide, N-(2-hydroxyethyl)-N-(3-cetyloxy-2-hydroxypropyl)amide of cetylic acid and N-docosanoyl-N-methyl-D-glucamine.

**5.** Composition according to any one of the preceding claims, **characterized in that** the ceramide-type compound (s) are present in concentrations ranging from 0.0001 to 20% by weight relative to the total weight of the composition and preferably from 0.001 to 10% by weight and more preferably between 0.005 and 3% by weight.

**6.** Composition according to any one of the preceding claims, **characterized in that** the liquid fatty alcohols are chosen from lauryl alcohol, myristyl alcohol, isomyristyl alcohol, isostearyl alcohol, isocetyl alcohol, isoarachidyl alcohol, 2-octyldodecanol, 2-butyloctanol and oleyl alcohol, and mixtures thereof.

**7.** Composition according to the preceding claim, **characterized in that** the fatty alcohol is chosen from isostearyl alcohol and isocetyl alcohol.

**8.** Composition according to any one of the preceding claims, **characterized in that** the concentration of liquid fatty alcohols varies between 0.5% and 10% by weight approximately relative to the total weight of the composition,

and preferably between 1 and 10% approximately and more preferably still between 1.5 and 3% by weight.

**9.** Composition according to any one of the preceding claims, **characterized in that** said cationic surfactant is chosen from:

A) the quaternary ammonium salts of the following general formula (IV):

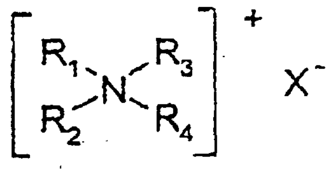

(IV)

in which X is an anion chosen from the group comprising halides (chloride, bromide or iodide) or ($C_2$-$C_6$)alkyl sulphates, more particularly methyl sulphate, phosphates, alkyl or alkylaryl sulphonates, anions derived from an organic acid such as acetate or lactate, and

i) the radicals $R_1$ to $R_3$, which may be identical or different, represent a linear or branched aliphatic radical comprising from 1 to 4 carbon atoms, or an aromatic radical such as aryl or alkylaryl. The aliphatic radicals may comprise heteroatoms such as in particular oxygen, nitrogen, sulphur or halogens, $R_4$ denotes a linear or branched alkyl radical comprising from 20 to 30 carbon atoms.

ii) the radicals $R_1$ and $R_2$, which may be identical or different, represent a linear or branched aliphatic radical comprising from 1 to 4 carbon atoms, or an aromatic radical such as aryl or alkylaryl. The aliphatic radicals may comprise heteroatoms such as in particular oxygen, nitrogen, sulphur or halogens. The aliphatic radicals are for example chosen from alkyl, alkoxy, alkylamide and hydroxyalkyl radicals comprising from about 1 to 4 carbon atoms;

$R_3$ and $R_4$, which are identical or different, denote a linear or branched alkyl radical comprising from 12 to 30 carbon atoms, said radical comprising at least one ester or amide functional group.

$R_3$ and $R_4$ are in particular chosen from ($C_{12}$-$C_{22}$) alkylamido ($C_2$-$C_6$) alkyl and ($C_{12}$-$C_{22}$) alkyl acetate radicals.

B) - the quaternary ammonium salts of imidazolinium, such as for example that of the following formula (V):

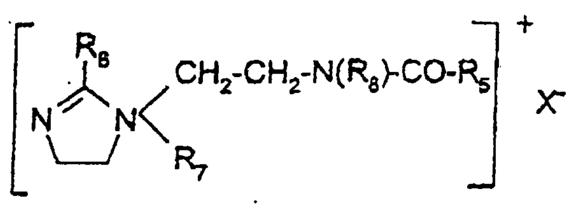

(V)

in which $R_5$ represents an alkenyl or alkyl radical comprising from 8 to 30 carbon atoms which are for example derived from tallow fatty acids, $R_6$ represents a hydrogen atom, a $C_1$-$C_4$ alkyl radical or an alkenyl or alkyl radical comprising from 8 to 30 carbon atoms, $R_7$ represents a $C_1$-$C_4$ alkyl radical, $R_8$ represents a hydrogen atom or a $C_1$-$C_4$ alkyl radical, X is an anion chosen from the group including halides, phosphates, acetates, lactates, alkyl sulphates and alkyl or alkylaryl sulphonates.

C) - the quaternary diammonium salts of formula (VI):

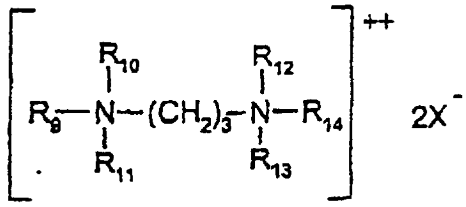

(VI)

in which $R_9$ denotes an aliphatic radical comprising from about 16 to 30 carbon atoms, $R_{10}$, $R_{11}$, $R_{12}$, $R_{13}$ and $R_{14}$, which are identical or different, are chosen from hydrogen or an alkyl radical comprising from 1 to 4 carbon atoms, and X is an anion chosen from the group comprising halides, acetates, phosphates, nitrates and methyl sulphates. Such quaternary diammonium salts comprise in particular propanetallowdiammonium dichloride. D) - the quaternary ammonium salts containing at least one ester functional group of the following formula (VII):

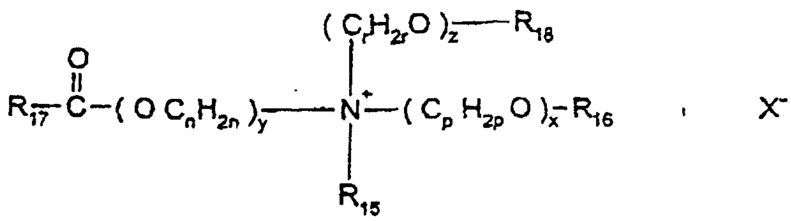

(VII)

in which:

- $R_{15}$ is chosen from $C_1$-$C_6$ alkyl radicals and $C_1$-$C_6$ hydroxyalkyl or dihydroxyalkyl radicals;
- $R_{16}$ is chosen from:

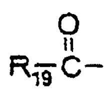

- the radical
- the linear or branched, saturated or unsaturated, $C_1$-$C_{22}$ hydrocarbon radicals $R_{20}$,
- the hydrogen atom,
- $R_{18}$ is chosen from:

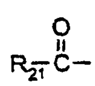

- the radical
- the linear or branched, saturated or unsaturated, $C_1$-$C_6$ hydrocarbon radicals $R_{22}$,
- the hydrogen atom,
- $R_{17}$, $R_{19}$ and $R_{21}$, which are identical or different, are chosen from linear or branched, saturated or unsaturated, $C_7$-$C_{21}$ hydrocarbon radicals;
- n, p and r, which are identical or different, are integers having values from 2 to 6;
- y is an integer having a value from 1 to 10;
- x and z, which are identical or different, are integers having values from 0 to 10;
- $X^-$ is an organic or inorganic, simple or complex anion;

with the proviso that the sum x + y + z has a value from 1 to 15, that when x has a value of 0, then $R_{16}$ denotes $R_{20}$, and that when z has a value of 0, then $R_{18}$ denotes $R_{22}$.

**10.** Composition according to any one of the preceding claims, **characterized in that** said cationic surfactant is chosen from behenyltrimethylammonium salts, stearamidopropyl dimethyl (myristyl acetate) ammonium salts, Quaternium-27 and Quaternium-83.

**11.** Composition according to any one of the preceding claims, **characterized in that** said cationic surfactant is present in concentrations ranging from 0.2 to 10% by weight relative to the total weight of the composition and preferably from 0.5 to 5% by weight and more preferably between 1 and 3.5% by weight.

**12.** Composition according to any one of the preceding claims, **characterized in that** the cosmetically acceptable medium consists of water or a mixture of water and at least one cosmetically acceptable solvent.

**13.** Composition according to Claim 12, **characterized in that** the cosmetically acceptable solvents are chosen from the group consisting of monoalcohols, polyalcohols, glcyol ethers and mixtures thereof.

**14.** Composition according to any one of Claims 1 to 13, **characterized in that** it comprises, in addition, at least one additive chosen from thickeners, perfumes, pearlescent agents, preservatives, sunscreens, anionic or nonionic or cationic or amphoteric polymers, proteins, protein hydrolysates, linear or branched chain $C_{16}$-$C_{40}$ fatty acids such as 18-methyleicosanoic acid, hydroxy acids, vitamins, panthenol and fatty esters.

**15.** Composition according to any one of Claims 1 to 14, **characterized in that** it is provided in the form of a shampoo, composition for permanent waving, straightening, dyeing or bleaching the hair, or washing composition for the skin.

**16.** Use of a composition as defined in any one of Claims 1 to 15, as or for the manufacture of a composition to be applied before or after any hair treatment such as shampooing, dyeing or bleaching, permanent waving or hair straightening, a rinse-out composition to be applied between the two stages of permanent waving or hair straightening, or a conditioner.

**17.** Method for treating keratinous materials, such as hair, **characterized in that** it consists in applying to said materials a cosmetic composition according to one of Claims 1 to 15, and then in optionally rinsing with water.

**18.** Use of a composition as defined in any one of Claims 1 to 15 for protecting keratinous materials, in particular the hair, from physical or chemical attacks.